Europäisches Patentamt

European Patent Office

Office européen des brevets

(19)

(11) Publication number: 0 248 465 B1

(12) EUROPEAN PATENT SPECIFICATION

(45) Date of publication of patent specification: 10.04.91    (51) Int. Cl.⁵: **C07C  65/05**, C07C 51/41,
           C10M 129/54

(21) Application number: 87200906.3

(22) Date of filing: 14.05.87

(54) **Process for the preparation of a basic salt, such a salt and lubricating oil compositions containing such a salt.**

(30) Priority: 06.06.86 GB 8613815

(43) Date of publication of application:
09.12.87 Bulletin 87/50

(45) Publication of the grant of the patent:
10.04.91 Bulletin 91/15

(84) Designated Contracting States:
BE DE FR GB IT NL

(56) References cited:
DE-A- 1 963 320
GB-A- 786 167
GB-A- 790 473

SOVIET INVENTIONS ILLUSTRATED, section
Ch, week C44, 10 Dec 1980, Derwent Publica-
tions Ltd., London HO6 (GB)

(73) Proprietor: SHELL INTERNATIONALE RE-
SEARCH MAATSCHAPPIJ B.V.
Carel van Bylandtlaan 30
NL-2596 HR Den Haag(NL)

(72) Inventor: Van Zon, Arie
Badhuisweg 3
NL-1031 CM Amsterdam(NL)
Inventor: Van Well, Rudolf Richard
Badhuisweg 3
NL-1031 CM Amsterdam(NL)
Inventor: De Lind van Wijngaarden, Gerhard
Badhuisweg 3
NL-1031 CM Amsterdam(NL)

(74) Representative: Aalbers, Onno et al
P.O. Box 302
NL-2501 CH The Hague(NL)

## Description

The present invention relates to a process for the preparation of a basic alkaline earth metal salt of an organic carboxylic acid, such a salt and to lubricating oil compositions containing such a salt.

The use of alkaline earth metal salts or organic carboxylic acids as additives for lubricating oil compositions is known. The said salts have a dispersant property so that they, when applied in such composition, ensure that the inside of engine cylinders remains clean and that deposition of carbonaceous products on pistons and in piston grooves is counteracted, so that piston-ring sticking is prevented.

It is also known to prepare basic (or overbased) alkaline earth metal salts of such acids. The overbasing provides an alkaline reserve which, when applied in lubricating oil compositions, reacts with and neutralises acidic compounds formed during the operation of the engine in which the composition is applied. Hence, sludge which may arise, is maintained dispersed due to the dispersant property of the salt while acids which would enhance sludge formation are neutralised.

In British patent specification No. 786,167, a process for the preparation of basic salts is described in which an organic acid is reacted with an excess of an alkaline earth metal oxide or hydroxide in a hydrocarbon solvent in the presence of an alcohol and subsequently carbon dioxide is passed through the reaction mixture to yield basic salts.

British patent specification No. 790,473 describes a process in which a colloidal dispersion of an alkaline earth metal carbonate in a $C_{1-6}$ alcohol is mixed with an organic carboxylic acid followed by removal of the alcohol, yielding a basic salt of the acid.

In the technical field there is a desire to use products with a basicity as high as possible, i.e. the relative proportion of the organic acid moiety in the basic salt is as low as possible. The reason for this is that the costs of the product are mainly incurred by the costs of the organic acid.

The basicity of the products prepared according to the prior art processes can approach a value of 10, the basicity index (BI) being defined as the equivalents ratio of the total of alkaline earth metal to the total of organic acid. However, it was found that when such prior art products have such a high BI, they tend to gel- thereby severely hindering the handleability of the products. The present invention provides a process which can yield products which have a very high BI and which show a considerably less pronounced gelling tendency.

Accordingly, the present invention relates to a process for the preparation of a basic alkaline earth metal salt of an organic carboxylic acid, which comprises:-

(a) preparing a mixture in a hydrocarbon solvent of one equivalent of the organic carboxylic acid and at least one equivalent of an alkaline earth metal hydroxide and/or oxide, and optionally a $C_{1-6}$ alcohol;

(b) introducing carbon dioxide into the mixture until at least 0.6 equivalent carbon dioxide per equivalent alkaline earth metal has been taken up;

(c) adding at least one further equivalent of alkaline earth metal hydroxide and/or oxide to the reaction mixture;

(d) resuming the introduction of carbon dioxide to the resulting mixture; and

(e) removing the aqueous layer and any residual solids.

Suitable organic carboxylic acids, which can be used in the present process include acids containing a benzene or naphthalene ring and an oil-solubilising radical or radicals having a total of at least 8, in particular at least 12 carbon atoms. Particularly preferred are alkyl salicylic acids having at least 10 carbon atoms in the alkyl group, in particular from 12 to 26 carbon atoms.

Other suitable acids include substituted or unsubstituted aliphatic or cycloaliphatic acids. Examples of these acids include naphthenic acids, and aliphatic acids having more than 8 carbon atoms, such as stearic, isostearic, palmitic, myristic, oleic and hydroxystearic acid, in particular tertiary carboxylic acids, sold by Shell under the trade name 'VERSATIC' acids.

The alkaline earth metal salts prepared include magnesium, calcium, strontium and barium salts. Preferably, the alkaline earth metal is magnesium or calcium.

The preparation of the mixture according to step a) of the present process can be carried out by any convenient way, e.g. by mixing the alkaline earth metal hydroxide and/or oxide with the alcohol and adding the acid, whether or not in the presence of the alcohol or the hydrocarbon solvent, to the resulting mixture. It is preferred to mix the acid and the alkaline earth metal hydroxide and/or oxide in a hydrocarbon solvent and subsequently add the alcohol. Before the addition of the alcohol the hydrocarbon solvent-containing mixture may be dried e.g. by azeotropic distillation or using molecular sieves.

The hydrocarbon solvent can be selected from a wide variety of solvents. Preferably it is an aromatic hydrocarbon or a hydrocarbon fraction rich in aromatics, such as gasoline. Suitable hydrocarbon solvents are benzene, toluene, xylene or mixtures thereof, xylene being particularly preferred. The alcohol used is preferably methanol. The amount of the solvent is not critical. Solvent:alcohol volume ratios up to 10 can suitably be applied, preference being given to ratios in the range from 6:1 to 2:1.

The amount of alkaline earth metal to be added in step a) must be at least 1 equivalent, thereby converting all the acid into the neutral alkaline earth metal salt. Preferably the amount of alkaline earth metal is more than 1 equivalent, so that the subsequent carbon dioxide supply results in basic compounds. Hence, the amount of alkaline earth metal oxide and/or hydroxide added in step a) is preferably from 5 to 15 equivalents per equivalent acid.

The temperature at which step a) is carried out is not critical. It may be ambient temperature or slightly elevated temperature. Suitably the temperature does not exceed 65°C; in particular a range of from 0 to 50°C is preferred.

Also in step b) the temperature is preferably below 65°C. The rate at which the carbon dioxide is introduced is advantageously from 0.05 to 0.5 equivalent carbon dioxide per equivalent acid per minute. The carbon dioxide introduction is conveniently carried out by passing carbon dioxide, or a mixture of carbon dioxide with an inert gas, such as air or nitrogen, through the reaction mixture under slightly elevated pressure. Higher pressures may be employed. Carbon dioxide will be absorbed in the reaction mixture and will react with the alkaline earth metal compounds present therein forming a basic complex salt of the organic acid salt and carbonate, hydroxide and/or oxide. The amount of carbon dioxide taken up in step b) is to a certain extent dependent on the amount of alkaline earth metal added in step a) of the present process. Suitably the relative amount of carbon dioxide is somewhat less than the relative amount of alkaline earth metal hydroxide or oxide.

Preferably the introduction of carbon dioxide in step b) is stopped after 0.6 to 0.9 equivalent carbon dioxide per equivalent alkaline earth metal has been taken up. Conveniently, this corresponds with 4 to 13 equivalent carbon dioxide per equivalent acid. Preferably, the uptake of carbon dioxide is at least 0.8, since although at lower uptakes products with high BI are obtained, these products tend to be rather viscous.

It has surprisingly been found that an ageing period between step b) and the addition of a further amount of alkaline earth metal hydroxide and/or oxide according to step c) is advantageous. This ageing period reduces the viscosity of the reaction mixture and has a beneficial effect on the viscosity properties of the end-product of the present process whereby the viscosity of the end-products in a hydrocarbon solvent or a lubricating oil does not become too high, so that the handleability of the end-products is warranted. Such an ageing period amounts suitably to at least 5 minutes. A maximum period is generally imposed by practical and/or economical reasons, and is generally below 2 hours. Preferably the period between steps b) and c) is from 10 to 45 minutes.

In step c) a further amount of alkaline earth metal hydroxide and/or oxide is added to the reaction mixture. The actual quantity thereof is determined by the desired basicity of the resulting end-product. Advantageously, when a BI of over 10 is desired, the amount of alkaline earth metal hydroxide and/or oxide, added in step c) is conveniently from 3 to 13 equivalent per equivalent acid.

Subsequently carbon dioxide introduction is resumed. The conditions are conveniently similar to those in the first carbonation step, i.e. step b), viz. a temperature below 65°C, and a rate of carbon dioxide of from 0.05 to 0.5 equivalent per equivalent acid per minute. The actual amount of carbon dioxide to be taken up in step d) is again dependent on the desired basicity and the amount of alkaline earth metal applied. Conveniently it is from 1 to 10 equivalent carbon dioxide per equivalent acid. Surprisingly, it has been found that it is advantageous to have an ageing period after this step before removal of solids and/or the alcohol-water layer obtained. The duration of this ageing period is preferably at least 3 hours and may extend to several days, e.g. 3 days. Conveniently the period does not exceed 19 hours. It has been found that after the ageing period the viscosity of the end-product has been reduced, its stability has been improved and its BI has been increased.

The reaction mixture at the end of step d) may be worked up by any method known in the art. It may be subjected to a centrifuging treatment to remove solids comprising unreacted alkaline earth metal hydroxide and/or oxide and/or non-colloidal alkaline earth metal carbonate, if any. The resulting solution may then be subjected to a liquid-phase separation. One liquid phase is an aqueous (water-alcohol) layer, the other one is the hydrocarbon solvent plus the basic salts dispersed therein. It is also possible to reverse the above operations.

The present process can be used for the preparation of basic salts having a wide variety of basicity indices. So, it would be possible to prepare basic salts having a BI of at least 1.1. The present process, however, is of particular value for preparing basic salts having a basicity index of from 10 to 20.

Preference is given to the salts in which the organic carboxylic acid is an alkylsalicylic acid in which the alkyl chain has at least 10 carbon atoms.

The basic salts are excellent dispersant additives in lubricating oils. Therefore the present invention facilitates preparation of lubricating oil compositions comprising a major amount of a lubricating base oil and a minor amount of a basic alkaline earth metal salt as described hereinbefore. The lubricating base oil will conveniently constitute more that 80%w of the composition. It can be selected from mineral lubricating oils of varying viscosities, but it also includes a synthetic lubricant, such as ester-type lubricant or a polyolefin-type fluid, or a vegetable oil, or a grease. Fuel oils which are used in marine diesel engines usually contain some sulphur compounds, resulting in the generation of sulphur oxides which pass into the lubricating oil. To neutralize the acidic compounds formed from these sulphur compounds a relatively high concentration of the basic salt is suitably employed in such marine lubricating oils. Preferably, these marine lubricating oil compositions contain from 5 to 15%w of basic alkaline earth metal salt. Lubricating oil compositions for road engines may contain lower concentrations. The amount of basic alkaline earth metal salt in these lubricating oil compositions is preferably from 0.01 to 5%w, in particular from 0.1 to 1.0%w.

Fuels, such as gasoline, kerosine, diesel fuel and gas oils, can also contain the above basic salts. The amount of these salts is similar to that in road engine lubricating oil compositions or lower; conveniently the amount is from 0.001 to 5%w, in particular from 0.01 to 1.0%w.

The lubricating oil composition can be prepared by mixing a concentrate containing up to 60%w of a basic salt as described above in a lubricating oil, with a lubricating base oil to give the desired concentration. The concentrate conveniently contains a stabiliser, which is selected from a variety of organic compounds, such as those described in British patent specification No. 818,315. These compcunds include mono- or polyhydric alcohols, alkyl amines and alkyl phenols.

The lubricating oil compositions may further contain a number of other additives, such as antioxidants, foam inhibitors, corrosion inhibitors, viscosity index improvers, and pour point depressants, as can be established by a person skilled in the art. The invention will be illustrated by means of the following Examples.

## EXAMPLE 1

Into a 3 l-reactor 1600 ml xylene was introduced together with 735 g(0.85 eq) $C_{14-18}$ alkylsalicyclic acid and 313 g (8.5 eq) calcium hydroxide. After 1 hour stirring at 40°C 435 ml methanol was added to the mixture. Subsequently at 50°C carbon dioxide was introduced at a rate of 0.12 eq/eq acid min. After 67 min the carbon dioxide supply was interrupted and it appeared that 8.0 eq $CO_2$/eq acid was taken up, corresponding with 0.8 eq $CO_2$/eq $Ca(OH)_2$. After a period of 15 min an additional amount of 219 g (5.9 eq) calcium hydroxide was added and after stirring for 1 hour the carbon dioxide introduction was resumed at the same conditions as before. After an uptake of 4.1 eq $CO_2$/eq acid (i.e. after 33 min) the carbon dioxide stream was stopped after which the reaction mixture was further stirred for 16 hours at 50°C. After settling of the two liquid phases, now present, the methanol-water layer was removed. From the xylene layer residual solids were removed by centrifugation. To 100 ml of the xylene layer 64 ml of a mineral lubricating base oil was added and subsequently the resulting mixture was subjected to vacuum distillation to remove the xylene and water present in the above-mentioned xylene layer, to yield a 10%m/m concentrate, based on calcium content. The basic calcium salt in this concentrate has a BI of 15.3. During the process no gelling tendency of the mixture in any step was noticed.

The viscosity of the xylene-layer at 40°C ($V_k40$) was 7.25 cS, and that of the concentrate at 100°C ($V_k100$) was 53.9 cS.

## EXAMPLE 2

The same procedure as described in Example 1 was carried out three times, with the exception that the carbonation reactions were interrupted and stopped in steps b) and d) after different uptakes of $CO_2$. No gelling occurred. The BI of the products was determined and also the $V_k40$ of the xylene layer and the $V_k100$ of the concentrate. Conditions and results are indicated in Table I.

## TABLE I

| Exp. No. | $CO_2$ uptake (eq. $CO_2$/eq acid) in step b) | in step d) | BI | $V_k40$ cS | $V_k100$ cS |
|---|---|---|---|---|---|
| 2 | 8.2 | 3.9 | 14.7 | 3.21 | 20.8 |
| 3 | 8.4 | 4.1 | 15.3 | 4.27 | 29.2 |
| 4 | 7.9 | 4.2 | 15.1 | 30.1 | 275 |

From these results it is apparent that in all cases products with high BI are obtained, but that the viscosities of the xylene layer and the concentrate increase considerably when the carbonation is interrupted after an uptake of 7.9 eq. $CO_2$/eq acid, corresponding with an uptake of 0.79 eq. $CO_2$/eq $Ca(OH)_2$.

## EXAMPLE 3

To show the influence of an ageing period after step b), the procedure according to Example 1 was repeated with the exception that the ageing period after step b) was varied between 0 and 75 min and that immediately after step d) the alcohol-water layer and solids were removed. The results are indicated in Table II.

## TABLE II

| Exp. No. | Ageing period min. | BI | $V_k40$ of xylene layer in cS | |
|---|---|---|---|---|
| | | | after 2 days (23°C) | after 7 days (23°C) |
| 5 | 0 | 15.4 | 8.35 | 9.09 |
| 6 | 15 | 15.1 | 3.54 | 3.61 |
| 7 | 30 | 15.0 | 2.80 | 2.83 |
| 8 | 75 | 13.0 | 1.91 | 1.91 |

From these results it is apparent that the ageing period has a beneficial effect on the viscosity of the end-product and also on the viscosity-stability. A decrease of BI takes place, but only significantly after about 30 minutes.

## EXAMPLE 4

To show the effect of the ageing period after step d) the procedures of Experiments 1-3 (see Examples 1 and 2) were repeated, but the ageing period after step d) was omitted. The results of these experiments are indicated in Table III. For comparison the results of experiments 1-3 are included in this Table.

## TABLE III

| Exp. No. | Ageing Period (hrs) after step d) | BI | $V_k40$ of xylene layer |
|---|---|---|---|
| 1 | 16 | 15.3 | 7.25 |
| 9 | - | 14.9 | 8.14 |
| 2 | 16 | 14.7 | 3.21 |
| 10 | - | 14.0 | 3.35 |
| 3 | 16 | 15.3 | 4.27 |
| 11 | - | 14.4 | 4.46 |

From these results it appears that the ageing period results in a lower viscosity and a higher BI of the end products.

Comparative Experiment A

In a 3 l-reactor 1340 ml of xylene was introduced together with 640 g of alkylsalicylic acid as in Example 1 (0.72 eq) and 400 g (10.8 eq) calcium hydroxide. After stirring for 1 hour at 40°C, 390 ml methanol was added to the reaction mixture, and at 50°C carbon dioxide was introduced at a rate of 0.12 eq $CO_2$/eq acid.min. After an uptake of 9.3 eq $CO_2$/eq acid the reaction mixture gelated to become a solid mass. A BI of this solid mass could not be determined, but in theory could only have reached a value of around 10-11.

**Claims**

1. Process for the preparation of a basic alkaline earth metal salt of an organic carboxylic acid, which comprises
   (a) preparing a mixture in a hydrocarbon solvent of one equivalent of the organic carboxylic acid and at least one equivalent of an alkaline earth metal hydroxide and/or oxide and optionally a $C_{1-6}$ alcohol;
   (b) introducing carbon dioxide into the mixture until at least 0.6 equivalent carbon dioxide per equivalent alkaline earth matel has been taken up;
   (c) adding at least one further equivalent of alkaline earth metal hydroxide and/or oxide to the reaction mixture;
   (d) resuming the introduction of carbon dioxide to the resulting mixture; and
   (e) removing the aqueous layer and any residual solids.

2. Process according to claim 1, in which the organic carboxylic acid is an alkylsalicylic acid having at least 10 carbon atoms in the alkyl group.

3. Process according to claim 1 or 2, in which the alkaline earth metal is calcium or magnesium.

4. Process according to any one of claims 1-3, in which the hydrocarbon solvent is selected from benzene, toluene, xylene, or a mixture thereof.

5. Process according to any one of claims 1-4, in which the $C_{1-6}$ alcohol is methanol.

6. Process according to any one of claims 1-5, in which the amount of alkaline earth metal hydroxide and/or oxide added in step (a) is from 5 to 15 equivalents per equivalent acid.

7. Process according to any one of claims 1-6, in which the introduction of carbon dioxide in step (b) is carried out at a temperature below 65° C and at a rate of from 0.05 to 0.5 equivalent carbon dioxide per equivalent acid per minute.

8. Process according to any one of claims 1-7, in which the reaction mixture obtained in step (b) is left for at least 5 minutes before the addition of a further amount of the alkaline earth metal hydroxide and/or oxide according to step (c).

9. Process according to any one of claims 1-8, in which the amount of alkaline earth metal hydroxide and/or oxide, added in step (c) is from 3 to 13 equivalents per equivalent acid.

10. Process according to any one of claims 1-9, in which an ageing period is introduced between steps d) and e).

11. Process according to claim 10, in which the duration of the ageing period is at least 3 hours.


**Revendications**

1. Procédé de préparation d'un sel basique de métal alcalino-terreux d'un acide carboxylique organique, qui comprend les étapes qui consistent à :
   (a) préparer un mélange, dans un solvant hydrocarboné, d'un équivalent de l'acide carboxylique organique et d'au moins un équivalent d'un hydroxyde et/ou oxyde de métal alcalino-terreux, et éventuellement d'un alcool en $C_1$-$C_6$;
   (b) introduire du gaz carbonique dans le mélange jusqu'à ce qu'au moins 0,6 équivalent de gaz carbonique par équivalent de métal alcalino-terreux ait été consommé;
   (c) ajouter au mélange réactionnel au moins un autre équivalent d'hydroxyde et/ou oxyde de métal alcalino-terreux;
   (d) reprendre l'introduction de gaz carbonique dans le mélange réactionnel; et
   (e) éliminer la phase aqueuse et tout solide résiduel.

2. Procédé selon la revendication 1, dans lequel l'acide carboxylique organique est un acide alkylsalicylique comportant au moins 10 atomes de carbone dans le groupe alkyle.

3. Procédé selon la revendication 1 ou 2, dans lequel le métal alcalino-terreux est le calcium ou le magnésium.

4. Procédé selon l'une quelconque des revendications 1-3, dans lequel le solvant hydrocarboné est choisi parmi le benzène, le tolène, le xylène ou un mélange de ceux-ci.

5. Procédé selon l'une quelconque des revendications 1-4, dans lequel l'alcool en $C_1$-$C_6$ est le méthanol.

6. Procédé selon l'une quelconque des revendications 1-5, dans lequel la quantité d'hydroxyde et/ou d'oxyde de métal alcalino-terreux ajoutée dans l'étape (a) est de 5 à 15 équivalents par équivalent d'acide.

7. Procédé selon l'une quelconque des revendications 1-6, dans lequel l'introduction de gaz carbonique dans l'étape (b) se fait à une température inférieure à 65° C et à un débit de 0,05 à 0,5 équivalent de gaz carbonique par équivalent d'acide par minute.

8. Procédé selon l'une quelconque des revendications 1-7, dans lequel le mélange réactionnel obtenu dans l'étape (b) est laissé pendant au moins 5 minutes avant l'addition d'une quantité supplémentaire d'hydroxyde et/ou d'oxyde de métal alcalino-terreux selon l'étape (c).

9. Procédé selon l'une quelconque des revendications 1-8, dans lequel la quantité d'hydroxyde et/ou d'oxyde de métal alcalino-terreux ajoutée dans l'étape (c) est de 3 à 13 équivalents par équivalent d'acide.

EP 0 248 465 B1

10. Procédé selon l'une quelconque des revendications 1-9, dans lequel une période de vieillissement est introduite entre les étapes (d) et (e).

11. Procédé selon la revendication 10, dans lequel la durée de la période de vieillissement est d'au moins 3 heures.


**Ansprüche**

1. Verfahren zur Herstellung eines basischen Erdalkalimetalls einer organischen Carbonsäure, welches
   a) das Herstellen einer Mischung in einem Kohlenwasserstofflösungsmittel eines Äquivalents der organischen Carbonsäure und mindestens eines Äquivalents eines Erdalkalimetallhydroxids und/oder -oxids und gegebenenfalls eines $C_{1-6}$-Alkohols;
   b) das Zuführenvon Kohlendioxid zu der Mischung, bis mindestens 0,6 Äquivalente Kohlendioxid pro Äquivalent Erdalkalimetall aufgenommen worden sind;
   c) das Zugeben mindestens eines weiteren Äquivalents von Erdalkalimetallhydroxid und/oder -oxid zur Reaktionsmischung;
   d) die Wiederaufnahme des Einführens von Kohlendioxid in die resultierende Mischung; und
   e) das Entfernen der wäßrigen Schicht und jeglicher Feststoffrückstände umfaßt.

2. Verfahren nach Anspruch 1, in welchem die organische Carbonsäure eine Alkylsalicylsäure mit mindestens 10 Kohlenstoffatomen in der Alkylgruppe ist.

3. Verfahren nach Anspruch 1 oder 2, in welchem das Erdalkalimetall Calcium oder Magnesium ist.

4. Verfahren nach einem der Ansprüche 1 bis 3, in welchem das Kohlenwasserstofflösungsmittel ausgewählt ist aus Benzol, Toluol, Xylol oder einer Mischung davon.

5. Verfahren nach einem der Ansprüche 1 bis 4, in welchem der $C_{1-6}$-Alkohol Methanol ist.

6. Verfahren nach einem der Ansprüche 1 bis 5, in welchem die in Stufe (a) zugegebene Menge an Erdalkalimetallhydroxid und/oder -oxid 5 bis 15 Äquivalente pro Äquivalent Säure beträgt.

7. Verfahren nach einem der Ansprüche 1 bis 6, in welchem das Einführen von Kohlendioxid in Stufe (b) bei einer Temperatur unter 65°C und bei einer Geschwindigkeit von 0,05 bis 0,5 Äquivalente Kohlendioxid pro Äquivalent Säure pro Minute durchgeführt wird.

8. Verfahren nach einem der Ansprüche 1 bis 7, in welchem die in Stufe (b) erhaltene Reaktionsmischung mindestens 5 Minuten lang stehen gelassen wird, bevor eine weitere Menge des Erdalkalimetallhydroxids und/oder -oxids gemäß Stufe (c) zugegeben wird.

9. Verfahren nach einem der Ansprüche 1 bis 8, in welchem die in Stufe (c) zugegebene Menge an Erdalkalimetallhydroxid und/oder -oxid 3 bis 13 Äquivalente pro Äquivalent Säure beträgt.

10. Verfahren nach einem der Ansprüche 1 bis 9, in welchem zwischen den Stufen d) und e) ein Alterungszeitraum eingefügt wird.

11. Verfahren nach Anspruch 10, in welchem die Dauer des Alterungszeitraums mindestens 3 Stunden beträgt.

8